(19) Europäisches Patentamt
European Patent Office
Office européen des brevets

(11) EP 1 976 850 B1

(12) **FASCICULE DE BREVET EUROPEEN**

(45) Date de publication et mention de la délivrance du brevet:
**15.08.2012 Bulletin 2012/33**

(21) Numéro de dépôt: **07718058.6**

(22) Date de dépôt: **19.01.2007**

(51) Int Cl.:
***C07D 401/14*** *(2006.01)* ***G01N 33/532*** *(2006.01)*
***A61K 49/10*** *(2006.01)*

(86) Numéro de dépôt international:
**PCT/FR2007/000109**

(87) Numéro de publication internationale:
**WO 2007/083036 (26.07.2007 Gazette 2007/30)**

(54) **NOUVEAUX LIGANDS ET COMPLEXES DE LANTHANIDES, ET LEUR UTILISATION EN TANT QU'AGENTS DE CONTRASTE**

NEUARTIGE LANTHANIDLIGANDEN UND -KOMPLEXE SOWIE IHRE ANWENDUNG ALS KONTRASTMITTEL

NOVEL LANTHANIDE LIGANDS AND COMPLEXES, AND USE THEREOF AS CONTRAST AGENTS

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK RS**

(30) Priorité: **20.01.2006 FR 0600531**

(43) Date de publication de la demande:
**08.10.2008 Bulletin 2008/41**

(73) Titulaire: **COMMISSARIAT A L'ENERGIE ATOMIQUE**
**75015 Paris (FR)**

(72) Inventeurs:
- **GATEAU, Christelle**
**F-38560 Champ sur Drac (FR)**
- **MAZZANTI, Marinella**
**F-38950 St Martin Le Vinoux (FR)**
- **NONAT, Aline**
**F-21380 EPAGNY (FR)**

(74) Mandataire: **Boubal, Denis Henri Jacques et al**
**Bureau Duthoit Legros Associés**
**96/98, Boulevard Carnot**
**B.P. 105**
**59027 Lille Cedex (FR)**

(56) Documents cités:
**WO-A-2005/021538 JP-A- 2005 187 546**

- **TAKALO H ET AL: "SYNTHESIS AND LUMINESCENCE OF NOVEL EUIII COMPLEXING AGENTS AND LABELS WITH 4-(PHENYLETHYNYL)PYRIDINE SUBUNITS" HELVETICA CHIMICA ACTA, VERLAG HELVETICA CHIMICA ACTA. BASEL, CH, vol. 79, no. 3, 1996, pages 789-802, XP001002467 ISSN: 0018-019X**
- **C. GATEAU ET AL.: "Solid-state and soltuion properties of the lanthanide complexes of a new nonadentate tripodal ligand derived from 1,4,7-triazacyclononane" DALTON TRANS., 2003, pages 2428-2433, XP002402552**
- **M. MAGERSTÄDT ET AL.: "Gd(DOTA): An alternative to Gd(DTPA) as a T1,2 relaxation agent for NMR imaging or spectroscopy" MAGNETIC RESONANCE IN MEDICINE, vol. 3, no. 5, 1986, pages 808-812, XP009073651**
- **A. NONAT ET AL.: "Lanthanide Complexes of a Picolinate Ligand Derived from 1,4,7-Triazacyclononane with Potential Application in Magnetic Resonance Imaging and Time-Resolved Luminescence Imaging" CHEM. EUR. J., vol. 12, 6 juin 2006 (2006-06-06), pages 7133-7150, XP002434539**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 976 850 B1

## Description

La présente invention concerne notamment de nouveaux ligands et complexes de lanthanides, et leur utilisation en tant qu'agents de contraste, pour l'imagerie par résonance magnétique et optique dans le domaine médical.

L'imagerie par résonance magnétique (IRM) est une technique puissante de diagnostic médical basée sur la RMN. Afin d'augmenter l'intensité du signal et la qualité des images obtenues par IRM, des agents de contrastes sont utilisés.

Les propriétés spectroscopiques et magnétiques uniques des ions lanthanides font de ces métaux et de leurs complexes des molécules idéales pour leur utilisation dans le domaine médical et biochimique, et notamment, en tant qu'agents de contraste pour l'imagerie par résonance magnétique et en tant que traceurs optiques.

Selon les règles émises par l' «International Union of Pure and Applied Chemistry » (IUPAC), on entend par lanthanide la série des éléments chimiques allant du Cérium (Z=58) au Lutécium (Z=71).

Alors que les complexes d'europium ($Eu^{III}$) et de terbium ($Tb^{III}$), avec leur luminescence à vie longue et leurs spectres d'émissions bien définis, sont souvent utilisés dans la conception de détecteurs, comme sondes spectroscopiques et luminescentes pour résoudre des problèmes structuraux et analytiques et en tant que systèmes imageurs à fluorescence, le gadolinium (III), avec un moment magnétique élevé (S=712) et une relaxation électronique lente, est un métal idéal pour la conception des agents relaxants pour l'imagerie de résonance magnétique (IRM).

La conception et la structure d'un ligand, fondamentales pour une utilisation dans le domaine médical, font souvent l'objet d'études dans le domaine de la chimie de coordination.

Les ligands poly(amino)carboxylates ont été particulièrement étudiés. En effet, leurs stabilité cinétique et thermodynamique élevées sont des propriétés essentielles pour éviter la toxicité *in vivo.*

Actuellement, tous les agents de contraste de $Gd^{III}$ proposés dans le commerce sont des complexes présentant un poids moléculaire bas et obtenus à partir des ligands octadentates poly(amino)carboxylates, tels que, notamment, le macrocycle acide 1,4,7,10-tetraazacyrlododecane-N,N',N",N"'-tetraacétique ($H_4$dota) et le composé acyclique acide diethylènetriamine-N,N',N",N"'-pentaacétique ($H_5$dtpa).

Cela étant, dans ces complexes, la relaxivité est plus faible que le maximum théorique possible, ceci est dû à une absence d'optimisation simultanée de tous les paramètres déterminants et responsables de l'augmentation de la relaxation.

Comme déjà mentionné précédemment, la propriété clé des agents de contraste est leur "relaxivité". La relaxivité est définie comme l'aptitude d'un complexe à augmenter la vitesse de relaxation des protons des molécules d'eau environnante. Les complexes paramagnétiques du gadolinium(III) se sont imposés comme agents de contraste du fait des propriétés électroniques et magnétiques particulières de cet ion.

Une plus grande relaxivité est, une caractéristique nécessaire et essentielle pour la prochaine génération d'agents de contraste pour l'imagerie par résonance magnétique (IRM).

Une relaxivité plus élevée peut être obtenue en présence d'un plus grand nombre de molécules d'eau, associées à une optimisation de la vitesse d'échange de ces molécules et à un temps de corrélation rotationnel et de relaxation électronique longs.

Le gadolinium est un métal très toxique sous la forme hydratée $[Gd(H_2O)_9]^{3+}$. Pour éviter toute toxicité *in vivo,* il doit être utilisé sous forme d'un complexe inerte et thermodynamiquement stable. De plus, le ligand qui complexe le métal doit laisser des sites de coordination libres pour qu'une ou plusieurs molécules d'eau puissent se lier au métal, augmentant ainsi sa relaxivité.

Récemment, des complexes de gadolinium des ligands présentant une structure « tripode » où « tétrapode » avec des « bras » de picolinate et présentant des propriétés de relaxation intéressantes ont été publiés.

Toutefois, et malgré le fait que des études ont été menées afin de comprendre les paramètres moléculaires responsables de la relaxivité, les mécanismes et les propriétés de coordination sous-jacents à la relaxation électronique des complexes de Gd(III) restent toujours très peu compris et connus. Ceci empêche et rend plus difficile la conception de nouveaux ligands présentant une relaxation électronique idéale qui devient particulièrement importante pour la nouvelle génération de complexes macromoléculaires avec un temps de corrélation rotationnel long.

Par ailleurs, la préparation de complexes de lanthanides stables et hautement émetteurs de lumière dans de l'eau exige la conception de ligands polydentates contenant des photosensibilisateurs capables de protéger le métal central des molécules d'eau du solvant afin d'éviter la désactivation non radiative des états excités du métal lanthanide par les oscillateurs O-H.

Le ligand acide N,N'-bis[(6-carboxypyridin-2-yl)methyl]-ethylènediamine-N,N'-diacétique (H4bpeda) avec une structure « tetrapode » conduit à des complexes nona-coordonés de gadolinium solubles dans l'eau et avec une molécule d'eau liée à l'ion gadolinium. Ce complexe présente une relaxivité eau/proton et une vitesse d'échange d'eau similaire, voire même un peu plus favorable, aux agents de contraste commercialisés et semble présenter la relaxation transversale électronique la plus rapide connue jusqu'à aujourd'hui.

Des propriétés de relaxation complètement différentes ont été observées dans le cas du ligand nona-dentate

hautement symétrique 1,4,7-tris[(6-carboxypyridin-2-yl)methyl]-1,4,7-triazacyclononane ($H_3$tpatcn).

Le ligand $H_3$tpatcn conduit à un complexe de gadolinium nonacoordoné hautement rigide. Ce complexe ne contient pas de molécules d'eau coordonées et présente une relaxivité particulièrement haute à champ bas. Une relaxation électronique de spin lente a été estimée à partir du profil de [Gd(tpatcn)] obtenu par dispersion par relaxation magnétique nucléaire (DRMN) en accord avec des études détaillées obtenues en Résonance Eléctronique Paramagnétique (RPE) montrant, pour le cas de ce complexe, la plus petite largeur entre bandes observée pour les chélates de gadolinium.

La relaxation électronique de spin à champ zéro (approx. 1500 ps) est la plus grande valeur obtenue à ce jour pour ce type de complexe (650 ps pour dota). La relaxation électronique lente de ce complexe a été attribuée à la sphère de coordination inhabituelle contenant six atomes donneurs azotés associés à une symétrie élévée.

Toutefois, et malgré les nombreuses études réalisées concernant la préparation des complexes hautement luminescents, les marqueurs luminescents comprenant des lanthanides, et commercialisés actuellement, restent rares de part leur difficulté de préparation.

En outre, les imageurs à fluorescence et les imageurs à résonance magnétique nucléaire, les deux principales techniques non destructives utilisées dans le domaine de la médecine, présentent quelques inconvénients, notamment une basse profondeur de pénétration dans les tissus pour le cas des imageurs à fluorescence et une basse sensibilité pour le cas des imageurs à résonance magnétique.

L'utilisation d'agents de contraste bifonctionnels pour les imageurs optiques et à résonance magnétique permet l'étude des mêmes structures biologiques à différentes résolutions et profondeurs.

Dû aux différentes exigences dans la conception des imageurs bimodales utilisés dans les deux techniques mentionnées ci-dessus, il y a aujourd'hui très peu d'exemples de molécules utilisées dans la synthèse de complexes de lanthanides qui présentent simultanément de bonnes propriétés magnétiques et optiques.

Le but de la présente invention est de proposer de nouveaux complexes de lanthanides, qui pallient aux inconvénients précités, particulièrement en ce qui concerne leur conception (imageurs bimodaux) et leur stabilité en milieu aqueux.

Un autre but de la présente invention est de proposer de nouveaux agents de contraste comprenant de nouvelles structures capables de complexer efficacement les lanthanides, et particulièrement le gadolinium et le terbium.

Un autre but de la présente invention est de proposer de nouveaux agents de contraste comprenant de nouvelles structures présentant une relaxation électronique lente.

Un autre but de la présente invention est de proposer de nouveaux agents de contraste comprenant de nouvelles structures présentant une relaxivité similaire à celle des agents de contrastes proposés dans le commerce.

Un autre but de la présente invention est de proposer de nouvelles structures capables, à la fois de complexer efficacement les lanthanides, avec une relaxivité similaire à celle des agents de contraste proposés dans le commerce, et possédants des propriétés optiques remarquables.

Un autre but de la présente invention est de proposer de nouvelles structures possédant des propriétés optiques remarquables.

D'autres buts et avantages de l'invention apparaîtront au cours de la description qui va suivre qui n'est donnée qu'à titre indicatif et qui n'a pas pour but de la limiter.

La présente invention concerne des ligands pour métaux, notamment des lanthanides, de formule générale (I) :

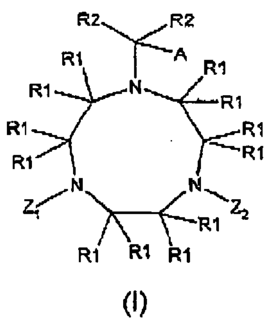

(I)

dans laquelle,

A, $R_1$, $R_2$, $Z_1$ et $Z_2$ sont comme définis dans la revendication 1.

La présente invention concerne également un complexe de coordination de formule générale :

$$[Ln(L)(H_2O)n]$$

dans laquelle L correspond à un ligand, selon la présente invention, et n est un entier entre 0 et 6.

La présente invention concerne par ailleurs un procédé de préparation des ligands et/ou complexes et/ou molécules d'intérêt caractérisé en ce qu'il comporte les étapes suivantes :

- fonctionnalisation de deux azotes du cycle d'un triazacyclononane par $Z_1$ et/ou $Z_2$ en présence de $Z_1$-LG et/ou $Z_2$-LG, notamment par substitution nucléophile, avec LG représentant un groupe labile,
- fonctionnalisation du triazacyclononane obtenu lors de l'étape précédente par $C(R_2)_2A$ en présence de LG-$C(R_2)_2A$, avec A représentant un ester d'alkyle ou d'aryle, et $R_2$ correspondant à un H.

La présente invention concerne en outre les ligands et les complexes greffés à une molécule d'intérêt biologique, ainsi que les agents de contraste et les compositions pharmaceutiques contenant au moins l'une de ces molécules.

L'invention sera mieux comprise à la lecture de la description suivante, accompagnée des dessins en annexe parmi lesquels :

- la figure 1 représente schématiquement la synthèse d'un ligand,
- la figure 2 représente une courbe de titration normalisée pour $H_3$bpatcn et bpatcn$^-$M.
- les figures 3a et 3b représentent respectivement les spectres d'émission de [Eu(bpatcn)] et [Tb(bpatcn)]$^-$ après excitation du ligand à 274 nm,
- la figure 4 représente le spectre d'absorption (......) de $H_3$bpatcn et le spectre d'excitation (-) de [Tb(bpatcn)] dans une solution tampon de Tris,
- la figure 5 représente schématiquement l'équilibre conformationnel de l'interconversion des différents stéréoisomères de [Ln(bpatcn)].

La présente invention concerne tout d'abord des ligands pour métaux, notamment des lanthanides, de formule générale (I) :

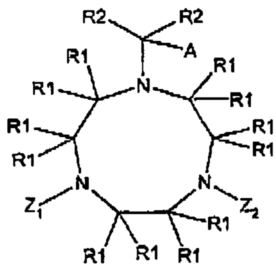

(I)

dans laquelle,
A, $R_1$, $R_2$, $Z_1$ et $Z_2$ sont comme définis à la revendication 1.

L'invention concerne également des complexes de coordination de formule générale :

$$[Ln(L)(H_2O)_n]$$

dans laquelle L correspond à un ligand tel que décrit dans la présente invention et n est un entier variant de 0 à 6, de préférence 1.

Selon un mode particulier de la présente invention, Ln est un lanthanide, à un degré d'oxydation trois choisi de préférence parmi le gadolinium, le terbium, l'europium, le neodinium, l'erbium et l'ytterbium,

Selon un autre mode particulier de la présente invention, les ligands et/ou les complexes tels que décrits dans la présente demande peuvent en outre être greffés à une molécule d'intérêt biologique.

Les molécules d'intérêt selon l'invention correspondent notamment aux biomolécules telles que les nucléotides, les polypeptides, les acides désoxyribonucléique (ADN) et ribonucléique (ARN), les anticorps, ou toute autre molécule active d'intérêt biologique et/ou médicinal. Il s'agit de toute molécule qu'un expérimentateur désire detecter au sein d'un organisme vivant, utilisant des techniques *in vivo* ou in *vitro,* et utilisant les propriétés magnétiques du métal et les propriétés optiques intrinsèques du métal ou l'éventuelle fluorescence apportée par le ligand.

Le greffage peut être réalisé par tout type de liaison ; ainsi pour un greffage durable il est souhaitable d'employer une liaison covalente alors que pour un greffage plus réversible des interactions hydrogènes peuvent être utilisées.

L'invention concerne également un procédé de préparation des ligands et/ou complexes et/ou molécules d'intérêt définis ci-dessus caractérisé en ce qu'il comporte les étapes suivantes :

(1) fonctionnalisation de deux azotes du cycle d'un triazacyclononane par $Z_1$ et/ou $Z_2$ en présence de $Z_1$-LG et/ou $Z_2$-LG, notamment par substitution nucléophile, avec LG représentant un groupe labile,
(2) fonctionnalisation du triazacyclononane obtenu en (1) par $C(R_2)_2A$ en présence de LG-$C(R_2)_2A$, avec A représentant un ester d'alkyle ou d'aryle, et $R_2$ individuellement et indépendamment tel que défini ci-dessus.

Selon un mode particulier de la présente invention, le groupement LG labile est choisi de préférence parmi un halogène, tel que Cl ou Br, un sulfonate, tel qu'un triflate, un tosylate ou un mesylate.

Le procédé peut comporter en outre au moins une étape de purification, réalisée notamment par chromatographie. L'ordre de réalisation des étapes peut être modifié, et la fonctionnalisation peut également commencer par réaction avec LG-$C(R_2)_2A$.

Une étape supplémentaire de greffage à une molécule d'intérêt est avantageuse. La molécule d'intérêt peut être greffée en de nombreux points sur le ligand ou le complexe par toute réaction adaptée à partir des fonctions disponibles et aménagées sur le complexe et notamment à partir de radicaux alkyles ou aryles présents sur la structure et tels que présentés ci-dessus. Il est préférable que le greffage soit effectué avec le ligand plutôt qu'avec le complexe.

Il est par exemple utile d'aménager sur la structure une fonction apte à créer une liaison peptidique pour ajouter un polypeptide. Il est ainsi possible de mettre à profit une fonction acide ou alcool présente sur un des radicaux alkyles pour permettre le greffage de dendrons ou de groupements susceptibles d'interagir avec une protéine telle que l'albumine, une base nucléotidique peut être utilisée pour un greffage sur un ADN ou un ARN.

Dans le cas de la préparation d'un complexe, le procédé comporte une étape d'incorporation d'un sel de lanthanide réalisée de préférence en milieu aqueux en présence d'un sel inorganique de lanthanide, tel que $LnCl_3.6H_2O$, et en ajustant le pH pour favoriser le piégeage du sel de lanthanide par le complexe.

L'invention concerne également les agents de contrastes contenant au moins un complexe et/ou ligand et/ou molécules d'intérêt décrits ci-dessus, ainsi que les agents de contrastes susceptibles d'être obtenus à partir des ligands et/ou complexes et/ou molécules d'intérêt définis ci-dessus.

L'invention correspond aussi à l'utilisation des ligands et/ou complexes de coordination décrits ci-dessus, greffés ou non à une molécule d'intérêt, pour la préparation d'un agent de contraste utilisables dans une méthode de diagnostic et notamment une méthode d'imagerie médicale.

De préférence cette méthode met à profit les propriétés magnétiques et/ou fluorescentes des complexes employés. Les méthodes concernées sont notamment l'imagerie par résonance magnétique (IRM), la microscopie de luminescence en temps résolu, et le transfert d'énergie (en Anglais, FRET Fluorescence résonance energy transfer).

L'application à l'imagerie par diffraction des rayons X et dans le développement de radiotraceurs est aussi envisagée.

L'invention correspond également aux compositions pharmaceutiques, contenant au moins un ligand et/ou un complexe, greffés ou non à une molécule d'intérêt et/ou un agent de contraste tels que définis précédemment, utilisables dans une méthode de diagnostic et notamment une méthode d'imagerie médicale.

En outre la composition peut contenir tout excipient, tel qu'une solution aqueuse toléré par l'organisme humain et/ou animal, connu de l'homme du métier et utilisable pour véhiculer les agents de contraste.

L'invention concerne aussi l'utilisation d'au moins un ligand et/ou un complexe et/ou d'une molécules d'intérêt et/ou un agent de contrastes tels que définis ci-dessus, pour la préparation d'une composition pharmaceutique utilisable dans une méthode de diagnostic et notamment une méthode d'imagerie médicale.

Les méthodes de diagnostic concernées par l'invention sont les méthodes d'imagerie et plus particulièrement l'imagerie par résonance magnétique nucléaire et par fluorescence.

L'un des avantages de l'invention est que les ligands tels que définis possèdent une affinité forte pour les lanthanides par rapport aux sels que l'on trouve dans les milieux biologiques comme le calcium. Cette selectivité suggère une innocuité accrue des agents de contrastes lors d'une utilisation *in vivo* car elle évite une transmétallation avec des sels présents dans l'organisme tels que le $Ca^{2+}$.

La forte luminescence, associée à une solubilité dans de l'eau et à une stabilité physiologique, indique que le complexe $[Ln(L)H_2O_n]$, tel que $[Tb(bpatcn)(H_2O)]$, est adapté pour le développement des imageurs à luminescence pour les applications biomédicales, telles que les tests immunologiques à fluorescence.

Un autre avantage de l'invention est qu'il est possible de préparer des composés (avec de métaux différents) contenant une même molécule organique qui se distribue donc de la même manière dans les mêmes tissus mais qui peuvent etre detectés par des différents methodes. En effet la présence d'au moins un groupement aromatique ainsi que celle d'un sel de lanthanide contenant une molécule d'eau dans la première sphère de coordination assurent une double fonctionnalité à ces complexes. Une même molécule complexée à deux metaux différents (Gd ou Tb) permettra ainsi d'étudier la même structure biologique avec deux techniques différentes (IRM et microscopie de luminescence) et donc avec différentes résolutions et différentes profondeurs.

Par ailleurs, la fonctionnalisation aisée des ligands, tels que le $bpatcn^{3-}$, dans le but de préparer de macromolécules comme agents de contrastes avec des temps de corrélation plus longs, devrait permettre l'étude de l'influence de l'optimisation de la relaxation électronique sur la relaxivité des systèmes avec un poids moléculaire élevé.

De plus, les propriétés favorables de relaxation électronique observées pour les complexes de gadolinium par études RMN et RPE indiquent que, après greffage sur une macromolécule, une relaxivité supérieure à celle des agents de contrastes commerciaux pourrait être atteinte.

Enfin la fonctionnalisation facile des ligands devrait permettre d'obtenir des composés plus hydrophobes pour l'imagerie des cellules et de l'introduire dans des systèmes macromoléculaires.

A titre d'exemple, non limitatif, la préparation des ligands $H_3$bpatcn et des complexes Ln(bpatcn) est décrite ci-dessus.

Le ligand $H_3$bpatcn a été préparé, tel que schématisé à la figure 1, de la façon suivante:

Sous une atmosphère d'argon, le trihydrochlorure de 1,4,7-triazacyclononane (0,431 g, 1,81 mmol) et $K_2CO_3$ (1,05 g, 7,62 mmol) sont successivement additionnés à une solution d'ester éthylique de la 6-chloromethylpyridine-2-carboxylate (0,760 g, 3,81 mmol) dans l'acétonitrile anhydre (50 mL).

Après agitation à température ambiante pendant une heure, le mélange réactionnel est porté au reflux pendant 18 h. Les sels inorganiques sont filtrés et le solvant évaporé, puis, le produit obtenu est purifié par chromatographie sur colonne d'alumine activité III (50g, $CH_2Cl_2$/EtOH 100 à 9812) et le 1,4-bis[(6-carbethoxypyridin-2-yl)methyl]-1,4,7-triazacyclononane est obtenu sous la forme d'une huile jaune (0,193 g, 24%).

Ensuite, le chloroacetate d'éthyle (0,255 g, 2,08 mmol) et $K_2CO_3$ (0,288 g, 2,08 mmol) sont successivement additionnés à une solution de 1,4-bis[(6-carbethoxypyridin-2-yl)methyl]-1,4,7-triazacyclononane (0,860 g, 1,89 mmol) dans l'acétonitrile anhydre (60 mL).

Le mélange réactionnel est porté au reflux pendant la nuit. Après filtration et évaporation du solvant, le produit obtenu est purifié par chromatographie sur colonne d'alumine activité III (90g, $CH_2Cl_2$/EtOH 100 à 98/2) et le 1-carbethoxymethyl-4,7-bis[(6-carbethoxypyridin-2-yl)methyl]-1,4,7-triazacyclononane est obtenu sous la forme d'une huile jaune (0,568 g, 56%).

Une solution de KOH 1 M (6,5 mL) est additionnée à une solution de 1-carbethoxymethyl-4,7-bis[(6-carbethoxypyridin-2-yt)méthyl]-1,4,7-triazacyclononane (0,321 g, 0,570 mmol) dans de l'éthanol (10 mL). Le mélange réactionnel est porté au reflux pendant la nuit. Après évaporation du solvant, l'huile obtenue est dissoute dans de l'eau et le pH est ajusté à 1,5 par addition d'une solution aqueuse de HCl 1,2M.

Après évaporation lente de la solution, le ligand $H_3$bpatcn.2,5 KCl.2 HCl. 4 $H_2O$ est obtenu sous la forme de cristaux blancs (0,310g, 69%).

Les complexes Ln(bpatcn) peuvent être préparés de la façon suivante:

Une solution de $EuCl_3.6H_2O$ (0,13 mmol) dans l'eau (0,4 mL) est additionné à une solution de $H_3$bpatcn (0,15 mmol) dans l'eau (2 mL). Le pH de la solution obtenue est ajusté à 7,5 par addition d'une solution aqueuse de KOH 1M. Après évaporation de l'eau, le solide obtenu est dissout dans de l'éthanol (20 mL). La suspension obtenue est refroidie à 4°C pendant la nuit et KCl est enlevé par filtration. Après l'évaparation lente de la solution, le complexe [Eu(bpatcn)] est obtenu sous la forme d'un solide blanc microcristalline (66,3mg, 65%).

Les complexes [La(bpatcn)], [Lu(bpatcn)] et [Gd(bpatcn)] sont isolés selon la même procédure.

Les constantes de déprotonation de $H_3$bpatcn définies comme $K_{al} = [H_{6-i}L]^{3-i}/[H_{5-i}L]^{1-i}[H^+]$ ont été déterminées par titrage potentiométrique et présentent les valeurs suivantes : $pK_{a1}$ = 2,2(2), $pK_{a2}$ = 2,3(2), $pK_{a3}$ = 3,7(3), $pK_{a4}$ = 5,42 (3) et $pK_{a5}$ = 10,5(2) (0,1 M KCl, 298 K).

Les courbes de titrage pour le cas de $H_3$bpatcn et ses complexes de $Gd^{III}$ et $Ca^{II}$ sont représentées sur la Figure 2.

Les Spectres de résonance magnétique nucléaire (RMN) des ligands, à différents pH, montre des variations importantes du déplacement chimique (0,3-0,4 ppm) des deux protons méthylèniques (à côté de l'acide picolinique et carboxylique) pendant la cinquième (pH=10-13) et la quatrième protonations (pH=4,5-7). Pendant la deuxième et la troisième protonations (pH=1,5-4.5) des variations assez importantes ne sont observées que pour les protons méthylèniques proches des groupement picoliniques. Des variations importantes sont observées dans les déplacements chimiques des trois protons pyridyles ($H_3$ et, dans une moindre mesure, $H_4$, $H_5$, 0,3-0,2 ppm) après la deuxième et la troisième protonations.

Les courbes de protonation montrent que les deux premiers équivalents d'acide protonent de façon identique les différents types d'atomes d'azote du macrocycle ($pK_{a4}$=5,42(3), $pK_{a6}$=10,5(2)). Les deux équivalents suivants protonent les groupements carboxylates liés aux pyridines ($PK_{a2}$=2,3(2), $pK_{a3}$=3,71(3).

La valeur de $pK_{a1}$ (2,2(2)) est en adéquation avec la valeur trouvée pour la protonation du groupement carboxylate dans l'acide 1,4,7-triazacyclononane-N,N',N"-triacetique ($H_3$nota) ligand (2.88(2)), décrite par C. F. G. C. Geraldes, M. C. Alpoim, M. P. M. Marques, A. D. Sherry, M. Singh, Inorg. Chem. 1985, 24, 3876-3881.

La structure cristalline du ligand $H_3$bpatcn.2HCl protoné isolé à un pH-2 dans laquelle tous les acides carboxyliques et les deux azotes adjacents aux groupements picolinates du macrocycle sont protonés est également en accord avec l'attribution du $pK_{ai}$ à la protonation de l'acide carboxylique.

La courbe de protonation et les données structurales sont en accord avec une protonation partielle simultanée des trois azotes du macrocycle tel qu'observé pour le ligand $H_3$nota, suivie par la protonation des groupements carboxylases. La protonation de la troisième amine et des azotes des pyridines a lieu à un pH plus bas et le $pK_a$ associé n'a pas été déterminé.

Les deux valeurs de $pK_a$ les plus elevées sont semblables au $pK_a$ le plus élévé pour le cas de la triamine cyclique 1,4,7-triazacyclononane (10,42 and 6,82) et du ligand macrocycle triaza $H_3$nota (11,3(1) et 5,59(2)).

Les valeurs de $pK_{a2}$ et $pK_{a3}$ sont en adéquation avec les valeurs trouvées pour la protonation des groupements picolinates dans le ligand à structure « tripode » $H_3$tpaa ($H_3$tpaa = acide $\alpha$, $\alpha'$, $\alpha''$ nitrilotri(6-methyl-2-pyridinecarboxylic) ($pK_{a2}$ = 3.3(1), $pK_{a3}$ = 4.11(6)).

Alors que l'introduction des groupements pyridinecarboxylates est responsable d'une diminution du caractère basique du ligand $H_3$tpaa par rapport à $H_3$nta (acide nitrilotriethanoïque) et du ligand $H_4$bpeda par rapport à $H_4$edta, le ligand $H_3$bpatcn présente un type de protonation et une valeur de $pK_a$ très similaire au ligand parent $H_3$nota.

Les valeurs de pKa et log$\beta$ pour $H_4$bpdea et pour des ligands de la même famille sont montrées au tableau cidessous

| ligand | pKa | Log $\beta$GdL | log$\beta$CaL |
|---|---|---|---|
| $H_3$bpatcn[a] | 10,5(2); 5,42(3); 3,71(3); 2,3(2) ; 2,2(2) | 15,8(2) | 8,18(7) |
| $H_3$nota[b] | 11,3 ; 5,6 ; 2,88 | 13,7 | 8,92 |
| $H_4$bpdea[b] | 8,5(1); 5,2(2); 3,5(1); 2,9(1) | 15,1(3) | 9,4(1) |
| $H_4$edta[b] | 10,19; 6,13 ; 2,69 ; 2,60 | 17,4 | 10,5 |
| $H_3$nta[b] | 9,75 ; 2,64 ;1,57 | 11,4 | |
| $H_3$tpaa[b] | 4,11(6) ; 3,3(1) ; 2,5(2) | 10,2(2) | 8,5(2) |
| [a] le présent travail<br>[b] littérature | | | |

Les constantes de stabilité des complexes de $H_3$bpatcn de $Gd^{III}$ et de $Ca^{II}$ ont été calculées par titrage direct d'un mélange de 1 ;1 métal : $H_3$bpatcn ($5,10^{-4}$ M) dans une gamme de pH entre 2,5 et 8,5.

Les données de titrage peuvent être représentées par les équations suivantes :

$$Gd^{3+} + bpatcn^{3-} \leftrightarrows [Gd(bpatcn)] \quad \log K_{GdL} = 15,8(2) ;$$

$$Ca^{2+} + bpatcn^{3-} \leftrightarrows [Ca(bpatcn)]^{-} \log K_{CaL} = 8,18\ (7)$$

Les valeurs de pGd = 13,6 et pCa= 6,30 (pM étant définit pour un métal M comme $-\log[M]_{libre}$ sous certaines conditions, ici pH 7,4, $[M]_{total}$ = 1$\mu$M et $[bpatcn]_{total}$ = 10 $\mu$M), qui permettent une comparaison directe des stabilités des complexes dans des conditions physiologiques, suggèrent une bonne stabilité par rapport à l'agent de contraste commercial $[Gd(dtpabma)(H_2O)]^-$ ($\log K_{GdL}$ = 1,.85, pGd = 15,8, pCa = 6,39).

Tandis que les valeurs de pKa et, par conséquent, la basicité de $H_3$bpatcn et de $H_3$nota sont très similaires, la stabilité du complexe de gadolinium de $bpatcn^{3-}$ est sensiblement plus élevée que celle du complexe nota ($\log K_{GdL}$ = 13,7).

La présence additionnelle de deux atomes d'azote donneurs d'électrons du groupement pyridyl dans bpatcn a comme conséquence une augmentation de la stabilité (2,1 log units) du complexe de gadolinium.

La contribution de la 2-pyridyimethyl à la stabilité a été estimée à 2,6 unités de log pour le cas du complexe $Gd^{III}$ avec N,N'-bis(2-pyridinylmethyl)ethylenediamine-N,N'-diacetate.

Les résultats décrits dans la présente demande montrent que la pyridine, contribue sensiblement à la stabilité du complexe de gadolinium, même lorsqu'elle fait partie du groupement 6-methyl-2-pyridinecarboxylique.

Par ailleurs, la constante de stabilité du complexe de calcium de $bpatcn^{3-}$ est similaire à celle de $nota^{3-}$.Par

conséquent, les groupements donneurs N, c'est-à-dire, les groupements pyridyls dans $bpatcn^{3-}$, sont responsables de la sélectivité de $Gd^{III}$ vis à vis du $Ca^{II}$ dans des ligands tels que décrits ci-dessus. La sélectivité élevée du ligand pour le gadolinium par rapport à des métaux physiologiques est très importante pour l'application de ces complexes dans l'imagerie par résonance magnétique (IRM), car la libération du $Gd^{III}$ associée à la transmétallation *in vivo* est responsable de la toxicité des complexes de gadolinium.

Les spectres d'absorption de $bpatcn^{3-}$ et ses complexes de $Eu^{III}$ et $Tb^{III}$ montrent une bande intense à -36500 $cm^{-1}$ avec un coefficient d'absorption molaire de 9050 pour Eu et de 9100 pour Tb. Ces bandes ont été attribuées à une combinaison de transitions centrées $\pi \rightarrow \pi^*$ et $n \rightarrow \pi^*$ du ligand.

Les figures 3a et 3b montrent respectivement les spectres d'émission des solutions des complexes d'europium et de terbium à pH=7,4 (obtenus après excitation à 273 nm) et les transitions habituelles et typiques $^5D_0 \rightarrow {}^7F_J$ et $^5D_4 \rightarrow {}^7F_J$ (J=0-6) des ions $Eu^{3+}$ and $Tb^{3+}$.

Les ions lanthanides Eu et Tb sont efficacement sensibilisés par le ligand $bpatcn^{3-}$ en ce qui concerne leurs propriétés de luminescence et, notamment, en ce qui concerne rémission dans la zone du visible.

Tel que montré à la figure 4, un transfert d'énergie efficace entre le métal et le ligand est suggéré par les similitudes entre les spectres d'excitation et d'absorption du chélate Tb.

Le rendement quantique d'émission du complexe de $[Tb(bpatcn)(H_2O)]$ ($\Phi$ = 43%) calculé par rapport à $[Tb(dpa)_3]^{3-}$ ($H_2dpa$ = acide dipicolinique) dans une solution tampon Tris 0,1M, avec une erreur expérimentale de 15%, présente une des valeurs les plus élevées trouvées jusqu'aujourd'hui et la valeur la plus élevée pour les complexes de terbium contenant une molécule d'eau coordonnée au métal central.

Le chromophore $bpatcn^{3-}$ sensibilise moins efficacement l'ion Eu. Le complexe a un rendement quantique moins élevé ($\Phi$ = 5%), proche du rendement quantique des sondes luminescentes trouvées dans le commerce.

La luminescence très intense de l'ion Tb est une conséquence d'un transfert d'énergie efficace du ligand vers le métal, et montre qu'il y a une protection efficace de l'ion métal vis-à-vis d'une désactivation non radiative malgré la présence d'une molécule d'eau coordonnée sur le métal.

Le temps de vie long de la luminescence observée pour le complexe de terbium dans l'eau (1,5 ms), exclut la présence d'un processus de désexcitation comprenant le retour d'énergie du métal dans son état excité vers le ligand.

L'inefficacité du processus non radiative de désexcitation (le plus important dans le cas du terbium), donne origine à un rendement quantique de luminescence élevé pour le complexe de terbium.

La comparaison de la luminescence dans l'eau et dans de l'eau deuterée montre que le processus de désexcitation non radiative provoqué par le solvant affecte beaucoup moins le rendement quantique du complexe de terbium que le rendement quantique du complexe d'europium.

Le tableau suivant montre que le chromophore bpatcn sensibilise le métal Tb très efficacement conduisant à une valeur de rendement quantique de $\Phi$=48%, beaucoup plus élevée que le rendement quantique des complexes de terbium existants dans les sondes commercialisées actuellement et décrit dans B. Alpha, V. Balzani, J.-M. Lehn, S. Perathoner, and N. Sabbatini, Angew. Chem. Int. Ed. Engl., 1987, 26, 1266 ; G. Mathis, Clin. Chem., 1993, 39, 195

| composé | $\lambda_{exc}$(nm) | s($M^{-1}cm^{-1}$) | $\tau_{H2O}$ (ms) | $\tau_{0,0}$ (ms) | $\Phi_{H2O}$ | $\Phi_{D2O}$ |
|---|---|---|---|---|---|---|
| bpatcn | 272 | 7850 | | | | |
| Eu(bpatcn) | 273 | 9050 | 0,542(4) | 1,67(4) | 0,05 | 0,12 |
| Tb(bpatcn) | 273 | 9100 | 1,49(2) | 2,46(7) | 0,43 | 0,48 |

Afin de déterminer la structure des complexes de lanthanides, les spectres de résonnance magnétique nucléaire (RMN) des complexes [Ln(bpatcn)] (Ln= La, Eu, Lu) ont été étudiés et comparés avec des résultats publiés pour les complexes $[Ln(dota)]^-$ et complexes de lanthanides avec des ligands contenant le noyau macrocyclique 1,4,7-triazacyclononane.

Les complexes de lanthanides avec le ligand $bpatcn^{3-}$ devraient présenter 24 signaux pour le spectre de RMN lorsque tous les atomes donneurs se trouvent coordonnés (symétrie $C_1$). La coordination rigide des ions de lanthanide peut donner origine à deux éléments structuraux indépendants de chiralité associés avec le cycle Ln-N-C-C-N et les angles de torsion des « bras » en suspension.

Le cycle peut présenter deux conformations énantiomériques ($\lambda\lambda\lambda$ et $\delta\delta\delta$) et les « bras » peuvent se trouver en forme d'hélice soit dans le sens des aiguilles d'une montre ($\Delta$) soit dans le sens contraire des aiguilles d'une montre (A).

En conséquence, deux paires d'énantiomères ($\Lambda(\lambda\lambda\lambda)/\Delta(\delta\delta\delta)$ ou $\Lambda(\delta\delta\delta)/\Delta(\lambda\lambda\lambda)$) de diasteroisomères peuvent être formées et l'interconversion par inversion du cycle (I) ou par rotation concertée des « bras » (II) peut avoir lieu, tel que montré à la figure 5.

La présence de deux paires de diasteroisomères avec une structure rigide prévoit qu'elle donne deux groupes de 24 signaux pour le $^1H$ RMN, tandis que l'existence d'un processus d'échange rapide entre les enantiomères donnerait

origine à une molécule avec une symétrie $C_8$ et présentant seulement 12 signaux pour le $^1$H RMN.

La disposition des « bras » chélatés dans une forme non hélicoïdale peut également se présenter dans ces complexes asymétriques et donnerait également origine à des isomères avec une symétrie $C_1$.

Des précédentes études sur des complexes de lanthanides du ligand trianionique hexadentate l'acide triaza 1,4,7-triazacyoclononane-N,N',N"-triacetique ($H_4$nota) dans de l'eau et ceux du ligand neutre hexadentate 1,4,7-tris (carbamoylmethyl)-1,4,7-triazacyclononane dans de l'acétonitrile, suggèrent la présence dans ces complexes d'un noyau flexible triaza avec une interconversion rapide entre les deux conformations de l'anneau à cinq membres Ln-N-C-C-N.

Le spectre RMN de proton du complexe de lanthane dans de l'eau à un pH=7,1 et à une température de 298 K, montre 12 signaux avec trois résonances pour les protons de la pyridine, deux résonnances (doublet) pour le groupement $CH_2$ proche de la pyridine, une résonnance pour les protons du groupement acétate et six larges résonnances superposées pour les protons de la moitié éthylénique du macrocycle.

Ces caractéristiques sont en accord avec la présence d'espèces présentant une symétrie $C_s$ dans lesquelles les deux « bras » de picolinate sont équivalents.

Le caractère diasterotopique du groupement $CH_2$ proche de la pyridine s'explique par le fait que la coordination des trois azotes du macrocycle présente un temps de vie long et par le fait que les azotes quaternaires adjacents présentent un caractère asymétrique.

Le déplacement chimique des protons méthylènes du groupement acétate et des protons germinales de la moitié éthylénique du macrocycle requiert une mobilité conformationelle des ligands en solution.

Les spectres de RMN dans de l'eau, à une température entre 298 et 278 K, et dans un mélange eau-méthanol, à une température entre 278 et 233 K, ne montrent que des signaux de plus en plus larges empêchant une interprétation du processus dynamique qui tient lieu.

Le spectre de $^1$H RMN du complexe bpatcn$^{3-}$ avec le métal diamagnétique Lu(III) dans une solution de $D_2O$ à pH=4,2 et à une température de 298K, montre seulement un groupe de 24 signaux, avec 6 résonnances pour les protons de la pyridine, 12 résonnances (6 axiales et 6 équatoriales) partiellement superposées pour les protons de la moitié éthylénique du macrocyle, et 6 résonnances pour les protons méthyléniques des « bras ».

Une expérience 2D-COSY associée à une expérience $^1$H-$^1$H NOESY a permis une attribution exacte des protons de la pyridine et des protons du méthylène des « bras » en suspension. Un fort effet NOE (de l'anglais Nuclear Overhauser Effect) est observé entre les protons $H_{8a/8b}$ et $H_{8a'/8b'}$ du $CH_2$ proches de la pyridine et les protons $H_9$ and $H_{9'}$ de la pyridine.

Les groupements $CH_2$-$CH_2$ du macrocycle forment un groupe complexe de multiplets, doublets de doublets et triplets qui n'ont pu être attribués que partiellement.

Le caractère diastereotopique des protons méthylènes (quartets AB) des « bras » et des protons du noyau macrocyclique résulte du caractère asymétrique des azotes quaternaires adjacents.

Ces caractéristiques sont en accord avec la présence d'une structure rigide avec une symétrie $C_1$ en solution dans laquelle le macrocycle et les « bras » restent liés à l'échelle du temps de la RMN. Les spectres de RMN restent inchangés dans une gamme de pH entre 4,2 et 9.

Dans le cas des complexes de lanthanides de dota$^{4-}$, deux isomères sont observés en solution à température ambiante et un comportement de coalescence est observé pour des températures supérieures 318K, associées à une rapide inter conversion des deux isomères.

En revanche, un seul isomère est observé à température ambiante pour le complexe [Lu(bpatcn)] et le spectre de $^1$H RMN obtenu dans $D_2O$ entre 278 et 343 K montre un spectre quasi inchangé dans cette gamme de température.

Des expériences EXSY bidimensionnelles ont été effectuées dans de l'eau à 298 K et à 343 K. Tandis qu'il n'a été détecté aucun echange à température ambiante, à 343 K le spectre bidimensionnel EXSY dans de l'eau montre des signaux croisés indiquant un échange entre deux espèces qui génère un plan de symétrie.

A 343 K, le spectre $^1$H RMN du complexe de Eu(III) de bpatcn$^{3-}$ dans une solution de $D_2O$ et à un pH=9,1, montre, tel que pour le cas de Lu, un seul groupe de 24 signaux étroits avec la même intensité en accord avec la présence des espèces avec une symétrie Ci à cette température.

Les signaux ont été complètement attribués à l'aide d'une expérience 2D-COSY associée à une expérience 1H-1H NOESY.

L'expérience EXSY bidimensionnelle effectuée à 343 K a montré 12 signaux croisés entre deux groupes de protons reliés par un plan de symétrie. Ceci peut être inféré à la présence en solution des paires d'énantiomères ($\Delta(\lambda\lambda\lambda)/\Lambda(\delta\delta\delta)$ ou $\Delta(\delta\delta\delta)/\Lambda(\lambda\lambda\lambda)$) en échange lent à cette température.

Ces résultats suggèrent que l'ion métallique se trouve entouré par les trois « bras » et que le macrocycle reste lié par les cinq azotes et les trois oxygènes, même à une température élevée pour le cas de l'europium et du lutétium, tel qu'observé précédemment pour les complexes de lanthanide du ligand symétrique nonadentate tpatcn. Par ailleurs, basée sur les études de RMN, une disposition similaire des atomes donneurs autour du métal central peut être anticipée pour les complexes de lanthanide de tpatcn$^{3-}$ et bpatcn$^{3-}$.

Une conformation similaire à été trouvée dans le cas d'un dérivé nonadentate 1,4,7-triazacyclononane avec

trois « bras » iminocarboxyliques.

Le même groupe de recherche (L. Tei, A. J. Blake, M. W. George, J. A. Weinstein, C. Wilson, M. Sohröder, Dalton Trans. 2003, 1693-1700.) a publié récemment la structure en état solide et en solution de complexes de lanthanides [Ln(L)($CH_3COO$)] d'un dérivé similaire, le 1,4,7-triazacyclononane heptadentate bis-anionique avec deux « bras » iminocarboxyliques. Tandis qu'une structure plus flexible en solution est observée pour ces complexes, les géométries de coordination à l'état solide sont similaires pour les deux ligands. Ceci est une conséquence de la présence du groupement 1,4,7 triazacyclononane qui oblige l'ion métallique Ln à prendre une structure prismatique trigonale.

Le spectre RMN proton du complexe [Eu(bpatcn)($H_2O$)] subit un fort élargissement quand la température est baissée, indiquant qu'un processus dynamique est ralenti. En dessous de 283K, les signaux deviennent plus étroits et, à 278 K, le spectre ressemble au spectre obtenu à 343 K, montrant les mêmes 24 signaux, un peu plus larges et légèrement déplacés. Les spectres de RMN ont été enregistrés tous les 10 K entre 343 K et 278 K permettant le suivi de l'évolution des signaux et l'attribution des signaux dans le spectre à la plus basse température.

Le complexe de gadolinium nona-coordonné de $bpatcn^{3-}$ montre une relaxivité à des champs imageurs semblable à celle présente dans les agents de contrastes actuellement commercialisés $[Gd(dota)(H_2O)]^-$ et $[Gd(dtpa)(H_2O)]^{2-}$ et semblable à celle observée dans le complexe nona-coordonné de $[Gd(bpdea)(H_2O)]^-$.

Malgré la présence de molécules d'eau coordonnées, l'architecture moléculaire de $bpatcn^{3-}$ est responsable de la luminescence de $Eu^{III}$ and $Tb^{III}$ et produit un complexe de terbium avec une luminescence à temps de vie long et avec un rendement quantique très élevé.

Sa luminescence intense, associée à sa solubilité et à sa stabilité dans l'eau, font du complexe [Tb(bpatcn)($H_2O$)] un excellent candidat pour son utilisation en tant que sonde luminescente dans le biomédical. Des calculs EPR ont permis d'estimer la relaxation électronique de ce complexe à champs imageurs. La relaxation électronique du complexe de Gd(bpatcn) est plus lente que celle trouvée dans les agents de contrastes commercialisés. Cette caractéristique devrait conduire à une plus grande relaxivité lorsque le complexe sera inclus dans un système macromoléculaire.

Le système décrit ci-dessus constitue une des rares sondes bifonctionnelles existantes et celle qui présente la luminescence la plus intense.

Naturellement, d'autres modes de mise en oeuvre, à la portée de l'homme de l'art, peuvent être envisagés sans pour autant sortir du cadre de l'invention.

## Revendications

1. Ligand pour métaux de formule générale (I)

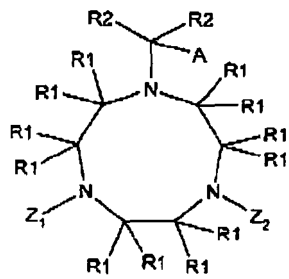

(I)

dans laquelle,
A correspond à un $CO_2H$,
$R_1$ et $R_2$ correspondent à un H,
$Z_1$ et $Z_2$, identiques, sont de formule générale:

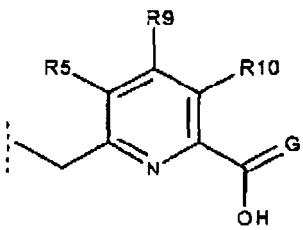

dans laquelle,
G représente un O,
$R_5$, $R_9$ et $R_{10}$ correspondent à un H.

2. Ligand selon la revendication 1, **caractérisé en ce qu'**il est un ligand pour lanthanides.

3. Complexes de coordination de formule générale :

$$[Ln(L)(H2O)_n]$$

dans laquelle Ln est un lanthanide, L correspond à un ligand selon l'une quelconque des revendications 1 à 2 et n est un entier entre 0 et 6.

4. Complexe selon la revendication 3, **caractérisé en ce que** n est égal à 1.

5. Complexe selon les revendications 3 ou 4, **caractérisé en ce que** le lanthanide est choisi parmi le gadolinium, le terbium, l'europium, le neodinium, l'erbium et l'ytterbium.

6. Complexe selon la revendication 4, **caractérisé en ce que** le lanthanide est choisi parmi le gadolinium, le terbium et l'europium.

7. Ligand selon les revendications 1 à 2 et/ou complexe selon l'une les revendications 3 à 6, **caractérisés en ce qu'**ils sont greffés à une biomolécule.

8. Agent de contraste **caractérisé par le fait qu'**il contient au moins un ligand selon les revendications 1 à 2 et/ou un complexe selon les revendications 3 à 6 et/ou une biomolécule.

9. Composition pharmaceutique **caractérisée en ce qu'**elle contient au moins un ligand selon les revendications 1 à 2 et/ou un complexe selon les revendications 4 à 6, greffés ou non sur une biomolécule, et/ou un agent de contraste selon la revendication 8.

## Claims

1. Ligand for metals having the general formula (I)

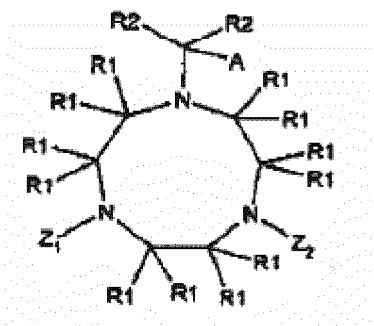

(I)

wherein,
A is $CO_2H$,
$R_1$ and $R_2$ are H,
$Z_1$ and $Z_2$ are identical and have the general formula:

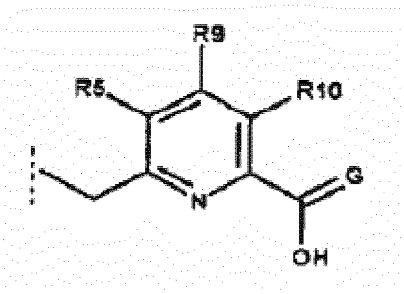

wherein,
G represents O,
$R_5$, $R_9$ and $R_{10}$ are H.

2. Ligand according to claim 1, **characterised in that** it is a ligand for lanthanides.

3. Coordination complexes having the general formula:

$$[Ln(L)(H2O)_n]$$

wherein Ln is a lanthanide, L is a ligand according to any of claims 1 to 2 and n is an integer between 0 and 6.

4. Complex according to claim 3, **characterised in that** n is equal to 1.

5. Complex according to claims 3 or 4, **characterised in that** the lanthanide is selected from gadolinium, terbium, europium, neodymium, erbium and ytterbium.

6. Complex according to claim 4, **characterised in that** the lanthanide is selected from gadolinium, terbium and europium.

7. Ligand according to claims 1 to 2 and/or complex according to any of claims 3 to 6, **characterised in that** they are grafted on a biomolecule.

8. Contrast agent **characterised in that** it contains at least one ligand according to claims 1 to 2 and/or a complex according to claims 3 to 6 and/or a biomolecule.

9. Pharmaceutical composition **characterised in that** it contains at least one ligand according to claims 1 to 2 and/or a complex according to claims 4 to 6, optionally grafted on a biomolecule, and/or a contrast agent according to claim 8.

**Patentansprüche**

1. Ligand für Metalle mit der allgemeinen Formel (I)

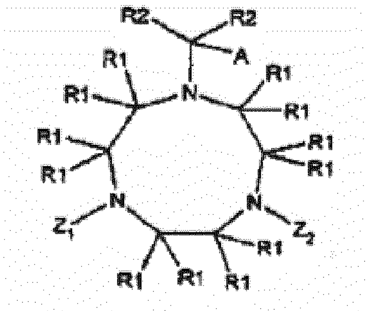

(I)

wobei:

A einem $CO_2H$ entspricht,
$R_1$ und $R_2$ einem H entsprechen,
$Z_1$ und $Z_2$, identisch, die folgende allgemeine Formel aufweisen:

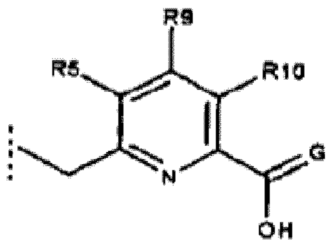

wobei:

G ein O darstellt,
$R_5$, $R_9$ und $R_{10}$ einem H entsprechen.

2. Ligand nach Anspruch 1, **dadurch gekennzeichnet, dass** er ein Ligand für Lanthanoide ist.

3. Koordinationskomplexe mit der allgemeinen Formel:

$[Ln(L)(H2O)_n]$

wobei Ln ein Lanthanoid ist, L einem Liganden nach einem der Ansprüche 1 bis 2 entspricht und n eine ganze Zahl zwischen 0 und 6 ist.

4. Komplex nach Anspruch 3, **dadurch gekennzeichnet, dass** n gleich 1 ist.

5. Komplex nach Ansprüche 3 oder 4, **dadurch gekennzeichnet, dass** das Lanthanoid ausgewählt ist aus Gadolinium, Terbium, Europium, Neodinium, Erbium und Ytterbium.

6. Komplex nach Anspruch 4, **dadurch gekennzeichnet, dass** das Lanthanoid ausgewählt ist aus Gadolinium, Terbium und Europium.

7. Ligand nach den Ansprüche 1 bis 2 und/oder Komplex nach einem der Ansprüche 3 bis 6, **dadurch gekennzeichnet, dass** sie auf ein Biomolekül gepfropft sind.

8. Kontrastmittel, **dadurch gekennzeichnet, dass** es mindestens einen Liganden nach den Ansprüche 1 bis 2 und/oder einen Komplex nach den Ansprüche 3 bis 6 und/oder ein Biomolekül umfasst.

**9.** Pharmazeutische Zusammensetzung, **dadurch gekennzeichnet, dass** sie mindestens einen Liganden nach den Ansprüche 1 bis 2 und/oder einen Komplex nach den Ansprüche 4 bis 6, die auf ein Biomolekül gepfropft sind oder nicht, und/oder ein Kontrastmittel nach Anspruch 8 umfasst.

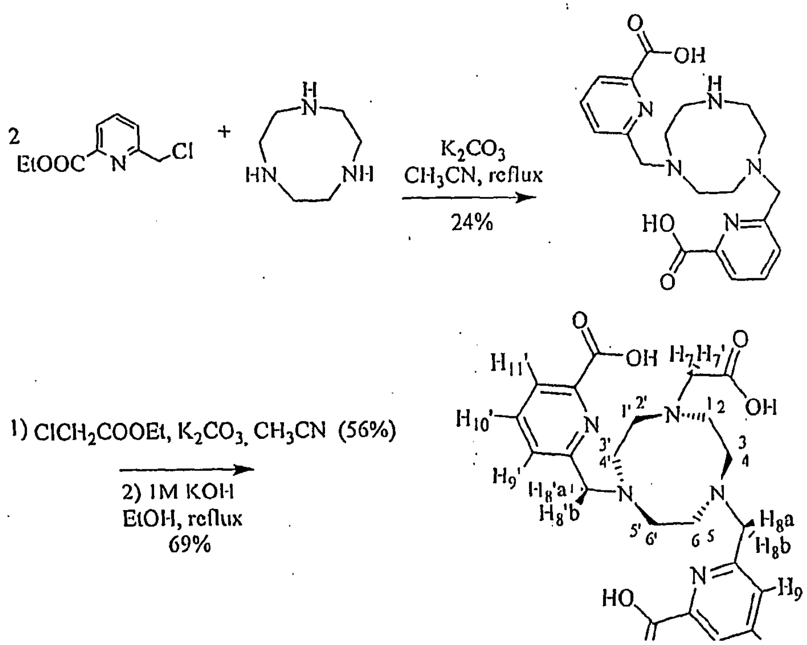
K2CO3
CH3CN, reflux
24%
1) ClCH2COOEt, K2CO3, CH3CN (56%)
2) 1M KOH
EtOH, reflux
69%

Fig.1

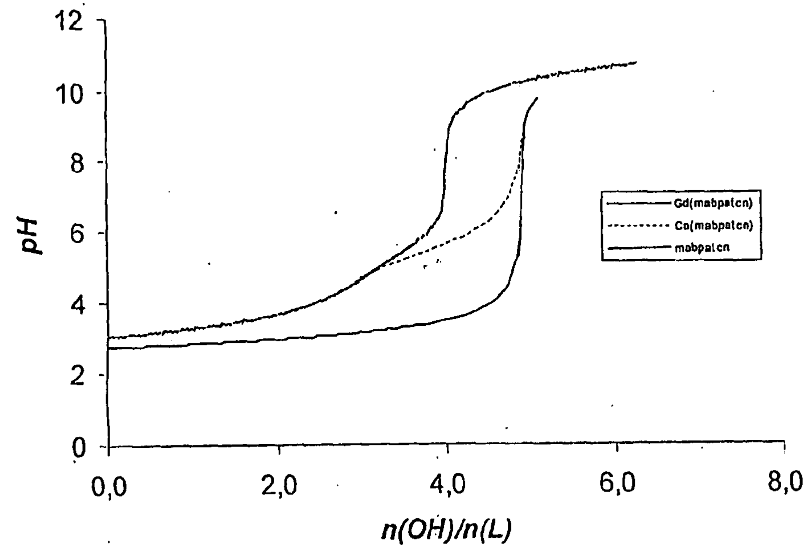
pH
n(OH)/n(L)
Gd(mabpatcn)
Ca(mabpatcn)
mabpatcn

Fig.2

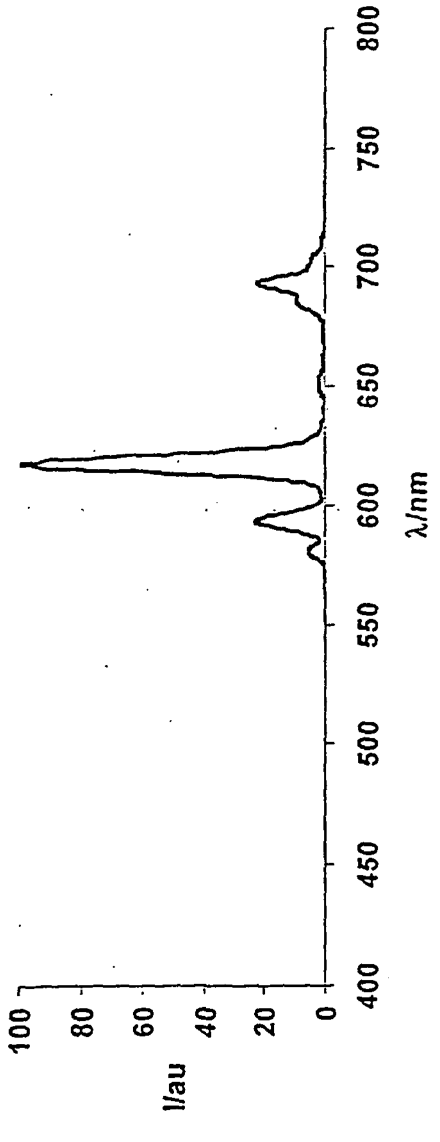
I/au
100
80
60
40
20
0
400
450
500
550
600
650
700
750
800
λ/nm

Fig.3a

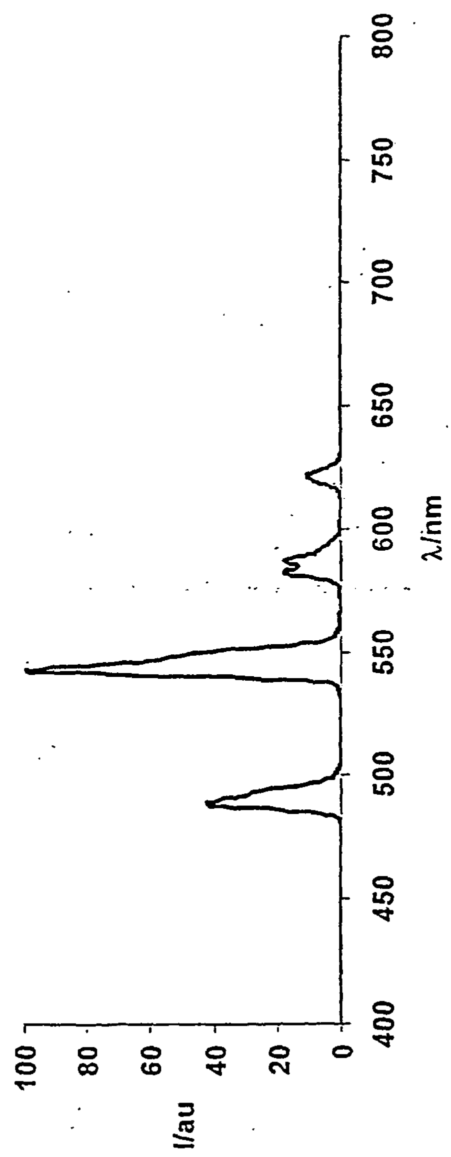
I/au
100
80
60
40
20
0
400
450
500
550
600
650
700
750
800
λ/nm

Fig.3b

Fig.4

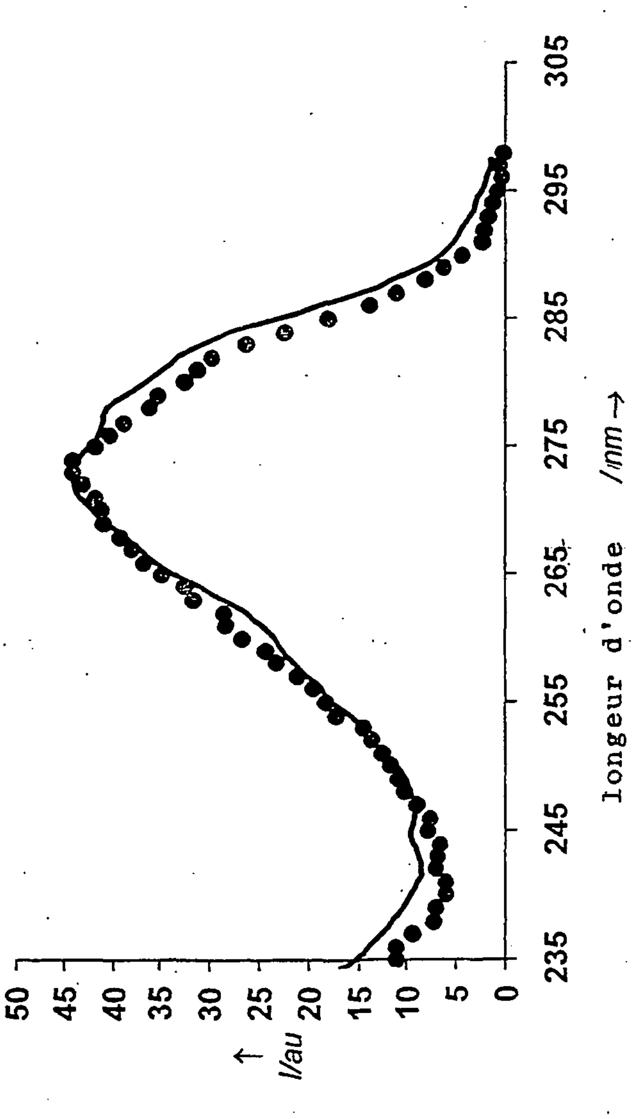

50 45 40 35 30 25 20 15 10 5 0
↑ I/au
235 245 255 265 275 285 295 305
longeur d'onde /nm →

Fig.5

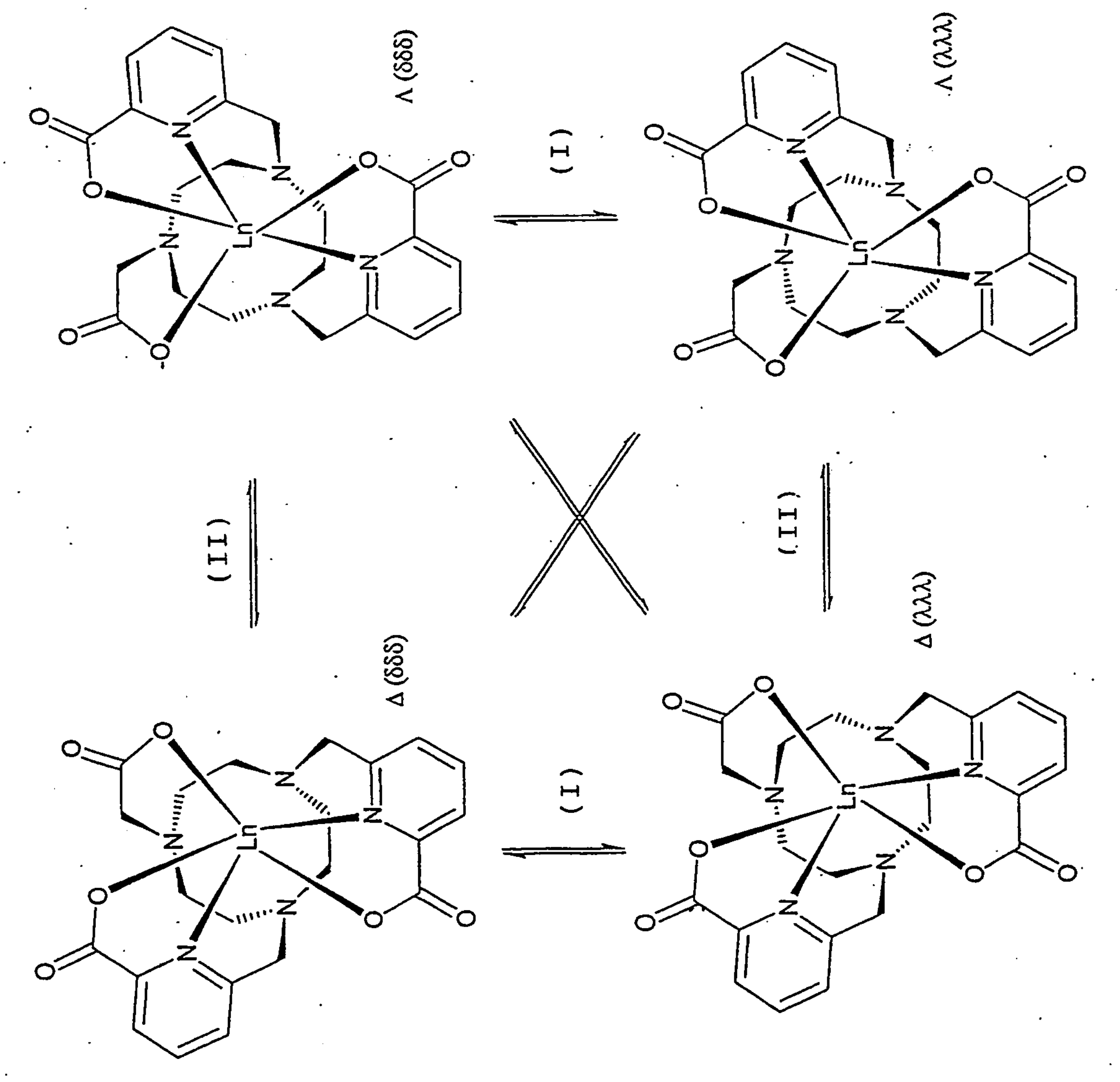

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **C. F. G. C. GERALDES ; M. C. ALPOIM ; M. P. M. MARQUES ; A. D. SHERRY ; M. SINGH.** *Inorg. Chem.,* 1985, vol. 24, 3876-3881
- **B. ALPHA ; V. BALZANI ; J.-M. LEHN ; S. PERATHONER ; N. SABBATINI.** *Angew. Chem.,* 1987, vol. 26, 1266
- **G. MATHIS.** *Clin. Chem.,* 1993, vol. 39, 195
- **L. TEI ; A. J. BLAKE ; M. W. GEORGE ; J. A. WEINSTEIN ; C. WILSON ; M. SOHRÖDER.** *Dalton Trans.,* 2003, 1693-1700